# EUROPEAN PATENT SPECIFICATION

(11) **EP 2 615 453 B1**
(45) Date of publication and mention of the grant of the patent: **22.03.2023**
(21) Application number: 11823763.5
(22) Date of filing: 07.09.2011
(51) Int. Cl.: G01N 21/84, G01N 33/487, G01N 21/27, G01N 21/25

(54) **MEASUREMENT APPARATUS**
MESSVORRICHTUNG
APPAREIL DE MESURE

(30) Priority: 10.09.2010 KR 20100088963
(43) Date of publication of application: 17.07.2013
(73) Proprietor: Ceragem Medisys Inc., Cheonan-si, Chungcheongnam-do 331-833 (KR)
(72) Inventor: YOON, Young Il, Cheonan-si Chungcheongnam-do 331-833 (KR); LEE, Jin Woo, Cheonan-si Chungcheongnam-do 331-833 (KR); CHOI, Jae Kyu, Cheonan-si Chungcheongnam-do 331-833 (KR)
(74) Representative: Dehns
(86) International application number: PCT/KR2011/006592
(87) International publication number: WO 2012/033330

(56) References cited:
- EP-A1- 1 033 569
- EP-A2- 0 416 587
- WO-A1-2010/059537
- CN-U- 201 508 314
- JP-A- H10 281 984
- JP-A- 2002 530 677
- JP-A- 2006 512 974
- JP-A- 2007 163 252
- KR-A- 20070 041 429
- US-A1- 2003 119 202
- US-B2- 7 118 713

## Description

### Technical Field

The present invention relates, in general, to measurement apparatuses and, more particularly, to an optical measurement apparatus which facilitates installation of a cartridge, is capable of automatic calibration, is small (portable), is easy to use, can rapidly determine a measurement result, and makes precise measurement possible.

In this specification, although a glycosylated hemoglobin measurement apparatus will be described as one example for the sake of understanding of the present invention, the measurement apparatus of the present invention may be used to measure albumin (microalbumin, U-Albumin), creatinine, C-reactive protein (CRP), D-dimer, etc.

### Background Art

Glycated hemoglobin or glycosylated hemoglobin refers to hemoglobin molecules of red blood cells carrying oxygen that is bound to glucose in blood. Since red blood cells are replaced with new ones after approximately 120 days, glycosylated hemoglobin indicates a blood glucose level with regard to a long term ranging from two to three months.

Hemoglobin in red blood cells of adults is formed of HbA (90%), HbA1 (7%), HbA2 (2%), HbF (0.5%), etc. HbA1 is subdivided into HbA1a, HbA1b and HbA1c (90%). Glycosylated hemoglobin typically refers to HbA1c (hemoglobin A1c). HbA1c refers to N-terminal valine of a hemoglobin β-chain that is ketoamine-linked to glucose.

Measurement of glycosylated hemoglobin is mainly used to check whether blood glucose management of a diabetic patient has been ordinarily carried out or not. Empty-stomach blood glucose measurement is allowed only under empty-stomach conditions and carries a risk of the measurement result being changed by several factors, e.g., pain or infection. On the other hand, glycosylated hemoglobin measurement does not require empty-stomach conditions, so it can be carried out any time. In addition, there are few factors which may affect the measurement result.

Recently, ADA (American Diabetes Association) announced a new diabetes diagnosis guide that recommends glycosylated hemoglobin measurement as a diabetes diagnosis method. Although the glycosylated hemoglobin measurement is not an entirely new technology, because standards therefor have not been defined, it has only been used as a follow-up test. Recently, a lot of clinical tests have been conducted, so the applicability of measurement results has markedly increased.

Ion-exchange chromatography, electrophoresis, high-performance liquid chromatography (HPLC), etc. are examples of the glycosylated hemoglobin measurement method.

However, in the case of the above-stated conventional techniques, a measurement operation is mainly conducted in a clinical pathology laboratory of a hospital, a cost is increased, a lot of time is required to conduct a subdividing operation, and the measurement result is excessively sensitive to temperature change. Furthermore, because the conventional techniques require specialized skills and an expensive large apparatus, it is very difficult for an ordinary person to use the techniques.

Meanwhile, point-of-care (PoC) technology is being spotlighted, since it can reduce expenses required to provide a measurement apparatus or conduct a measurement operation [by reducing the size of an apparatus or making a portable apparatus], can be easily handled, and can rapidly provide a measurement result. Here, the term point-of-care means carrying out a clinical pathological diagnosis in a place where a patient is; for example, in an emergency center, an outpatient department, a medical office, a patient's home, etc.

Therefore, to satisfy increased modern-day interest in personal health, and to meet requirements necessary to perform easy measurement of a person's body conditions, the technical development of a glycosylated hemoglobin measurement apparatus is keenly required so that not only a clinical pathological expert, but also an ordinary person can easily and quickly take a measurement of his or her body conditions and check the measurement result, at any time and in any place.

Meanwhile, although an optical glycosylated hemoglobin measurement method was proposed, it is difficult to accurately dispose of a cartridge, into which a sample has been injected, at a position corresponding to an optical unit. Further, a calibration operation for determining a deviation in the quantity of light and compensating for a measurement value must be periodically performed manually.

Given this, technical development of an optical glycosylated hemoglobin measurement apparatus is greatly required, which facilitates installation of a cartridge, is capable of automatic calibration, and makes precise measurement possible. US 7118713 B2 describes a tray assembly for an optical inspection apparatus. EP 1033569 A1 describes an optical inspection method and apparatus with removable inserts. EP 0416587 A2 describes a measuring apparatus for measuring the constituent concentration of a specimen. US 2003/119202 A1 describes an optical reflectance kit. WO 2010/059537 A1 relates to a readhead for an optical diagnostic system.

### Disclosure

### Technical Problem

Accordingly, the present invention has been made keeping in mind the above problems occurring in the prior art, and an object of the present invention is to provide a measurement apparatus which is easy to use, is more convenient for a user, and has a reduced size.

Another object of the present invention is to provide a measurement apparatus in which a white calibration kit is installed on a tray so that a calibration operation can be automatically carried out, and which is configured such that the white calibration kit can be replaced with a new one, thus increasing convenience of use, and preventing blood from being contaminated.

A further object of the present invention is to provide a measurement apparatus which uses a circular cartridge so that the cartridge can be easily installed in the measurement apparatus, and an optical unit can easily take the focus on a sample which has been injected into the cartridge.

Yet another object of the present invention is to provide a measurement apparatus which, using a display or a voice, can guide a user who is preparing a sample to be measured, thus further increasing convenience to use.

### Technical Solution

In order to accomplish the above objects, the present invention provides an optical measurement apparatus as claimed in claim 1.

A kit depression may be formed in a bottom surface of the tray seating depression, and a white calibration kit may be removably mounted into the kit depression, the white calibration kit being used to check a deviation in the quantity of light of the optical unit

The white calibration kit may have a white plate shape, with locking protrusions provided on opposite edges of the white calibration kit, each of the locking protrusions having a hook so that the locking protrusions are locked to the tray.

An upper surface of the white calibration kit mounted in the kit depression may be level with the bottom surface of the tray seating depression so that the cartridge can be seated onto the white calibration kit in the tray seating depression.

The optical unit may include: a light emitting part applying light to the sample contained in the fixed cartridge; and a light receiving part receiving light reflected from the sample by the light application of the light emitting part.

The light emitting part and the light receiving part of the optical unit may be installed in respective slots formed in the frame.

The light receiving part may be installed in a central portion of the frame, and the light emitting part may comprise a plurality of light sources arranged around the central portion of the frame.

When the tray is completely retracted into the main body, a sample injection hole of the cartridge seated on the tray may be disposed below the optical unit of the frame.

The control unit may calculate the measurement value of the sample when the cartridge installation detection unit detects a presence of the cartridge.

The control unit may instruct the optical unit to apply light to the white calibration kit mounted in the tray seating depression and receive light reflected therefrom and then check a deviation between the quantity of the received light and a reference quantity of light of the optical unit.

The control unit may compensate for the calculated measurement value of the sample using the deviation.

The optical measurement apparatus may further include a cartridge installation detection unit detecting whether the cartridge has been mounted on the tray when the tray is in the retracted state, wherein when the cartridge installation detection unit determines that the cartridge has not been mounted on the tray, the control unit checks the deviation.

When the cartridge has not been mounted on the tray and power is turned on and/or when the tray that has been extracted from the main body is retracted thereinto without the cartridge having been mounted, the control unit may check the deviation.

The control unit may guide a user conducting a sample preparation operation through a screen and/or a voice.

### Advantageous Effects

A measurement apparatus according to the present invention can precisely measure a sample and rapidly provide a measurement result.

Furthermore, the measurement apparatus of the present invention has a reduced size and is easy and convenient to use, thus enhancing the reliability.

In addition, even after a white calibration kit has been installed on a tray, a cartridge can be seated onto the tray. As soon as the operation of the measurement apparatus begins, automatic calibration is carried out using the white calibration kit that has been installed on the tray, whereby convenience of use can be enhanced.

Furthermore, because the cartridge has a circular shape, the cartridge can be placed onto the tray regardless of the orientation of the cartridge, and the optical unit can easily take focus on the sample that has been injected into the cartridge. Therefore, the efficiency of the measurement operation can be enhanced.

Moreover, the measurement apparatus can guide a user preparing the sample using a display or a voice, thus further increasing convenience to use.

### Description of Drawings

FIG. 1 is a perspective view illustrating the external shape of a glycosylated hemoglobin measurement apparatus, according to an embodiment of the present invention;
Fig. 2 is a perspective view showing the internal construction of the glycosylated hemoglobin measurement apparatus;
Fig. 3 is a perspective view showing an optical unit of the glycosylated hemoglobin measurement apparatus;
Fig. 4 is a sectional view illustrating the optical unit of Fig. 3;
Fig. 5 is a perspective view illustrating the operation of a photo-interrupter;
Fig. 6 is a perspective view of a white calibration kit to be placed on a tray;
Fig. 7 is a perspective view showing the tray that is in an extracted state;
Fig. 8 is a perspective view showing the tray that is in a retracted state;
Fig. 9 is a view showing screens which are guiding a sample preparation operation in the glycosylated hemoglobin measurement apparatus; and
Fig. 10 is a flowchart showing the operation of the glycosylated hemoglobin measurement apparatus.

### <Description of the Reference Numerals in the Drawings>

### Best Mode

Hereinafter, a glycosylated hemoglobin measurement apparatus according to an embodiment of the present invention will be described in detail with reference to the attached drawings.

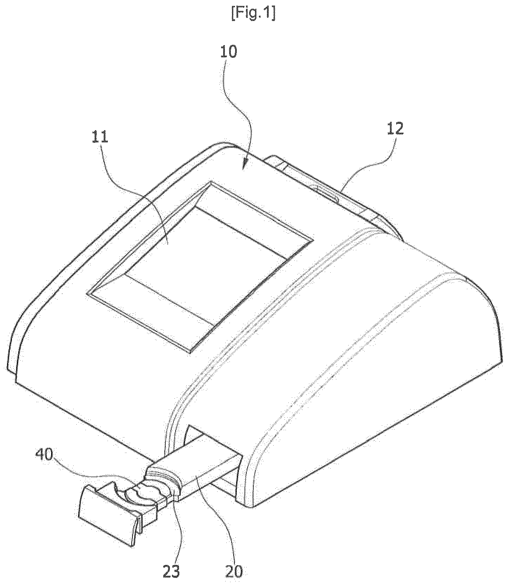

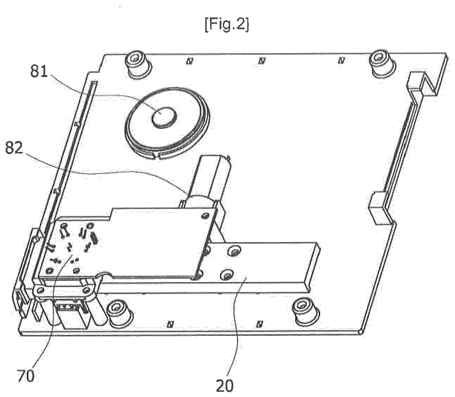

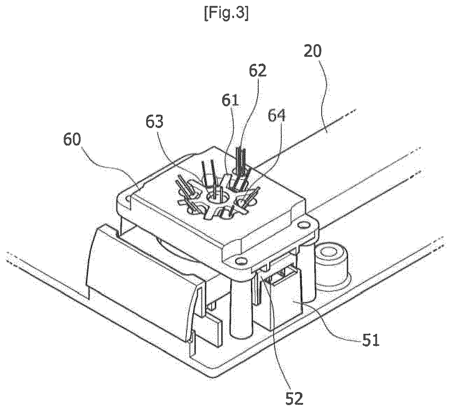

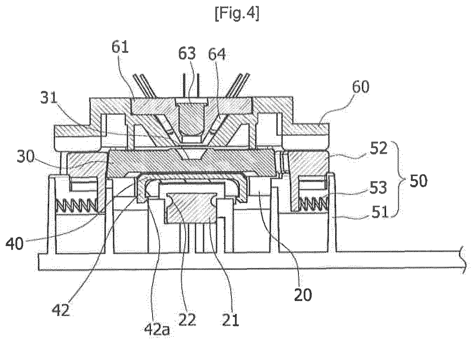

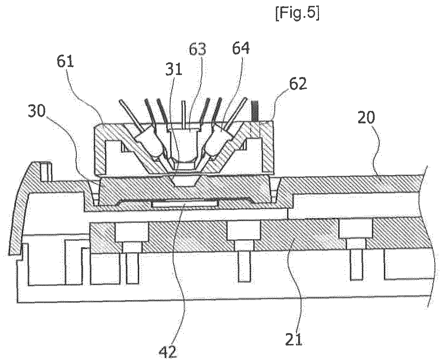

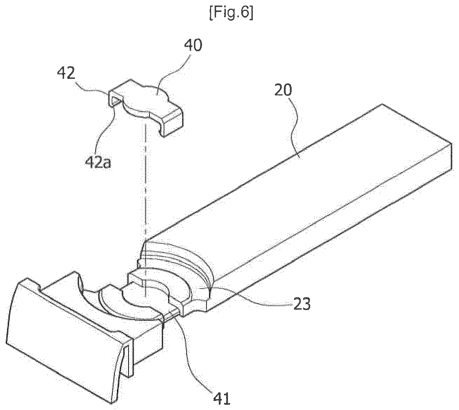

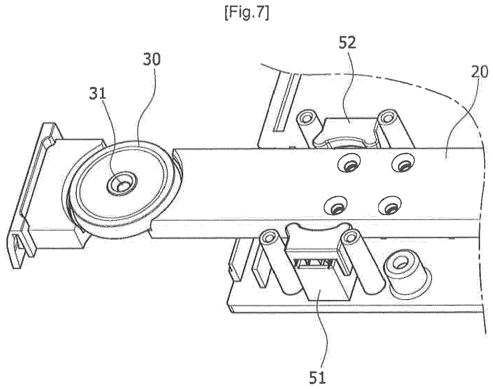

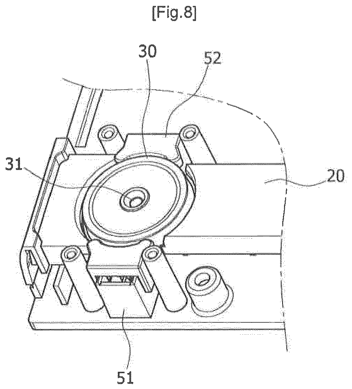

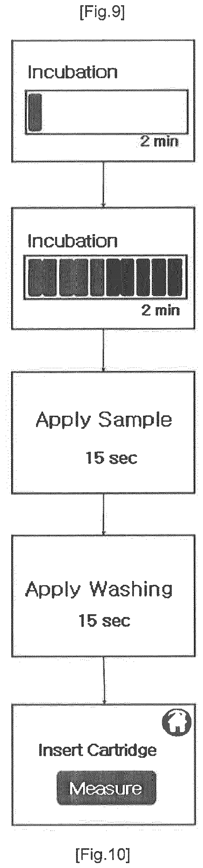

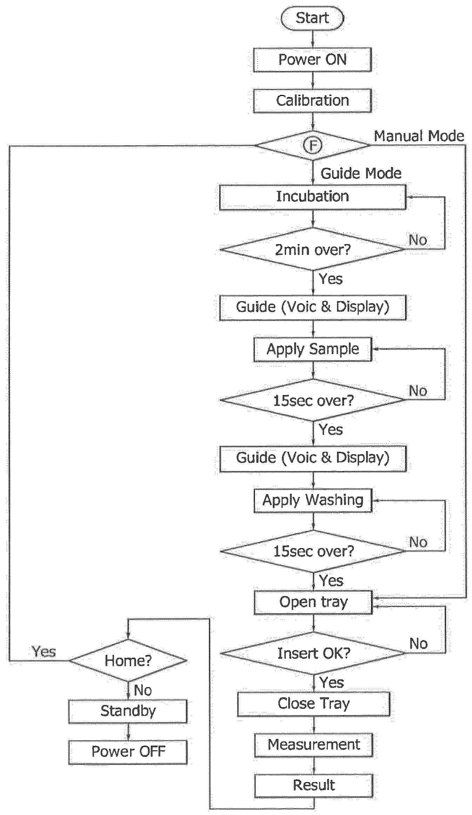

FIG. 1 is a perspective view illustrating the external shape of the glycosylated hemoglobin measurement apparatus, according to an embodiment of the present invention. Fig. 2 is a perspective view showing the internal construction of the glycosylated hemoglobin measurement apparatus. Fig. 3 is a perspective view showing an optical unit of the glycosylated hemoglobin measurement apparatus. Fig. 4 is a sectional view illustrating the optical unit of Fig. 3. Fig. 5 is a perspective view illustrating the operation of a photo-interrupter. Fig. 6 is a perspective view of a white calibration kit to be placed on a tray. Fig. 7 is a perspective view showing the tray that is in an extracted state. Fig. 8 is a perspective view showing the tray that is in a retracted state. Fig. 9 is a view showing screens which are guiding a sample preparation operation in the glycosylated hemoglobin measurement apparatus. Fig. 10 is a flowchart showing the operation of the glycosylated hemoglobin measurement apparatus.

Although it is not shown in the drawings, the glycosylated hemoglobin measurement apparatus of the present invention further includes a control unit, a memory unit, a data storage unit, a power unit, and various kinds of interfaces (RS-232, USB, Ethernet, etc.). Further, the apparatus may selectively further includes elements which are used in typical in-vitro diagnosis devices.

For example, a capillary tube, a capillary tube holder, a reagent (first reagent), a reagent tube, a washing solution (second reagent), a pipette, a tip, etc. can be selectively used as the elements (accessories) to measure glycosylated hemoglobin.

As shown in the drawings, the glycosylated hemoglobin measurement apparatus further includes a touch screen panel 11 (e.g., a touch LCD) which is provided on a front surface of a main body 10 of the apparatus to enable a user to input information (e.g., to select from a menu, to set configuration) in a touch screen input mode and to display a variety of information (e.g., a menu screen, a screen for guiding the sample preparation operation, measurement values). The apparatus further includes a printer 12 (e.g., a thermal printer) which is provided at a predetermined position in the main body 10 to print a variety of information (e.g., measurement values), and a speaker 81 which is installed at a predetermined position in the main body 10 to output various pieces of information (e.g., voices for guiding the sample preparation operation, measurement values) using a voice/sound or the like.

Furthermore, the glycosylated hemoglobin measurement apparatus of the present invention further includes a tray 20 onto which a circular cartridge 30, into which a sample (e.g., a mixed solution of biomaterial, such as blood, and reagent) has been injected, is placed (seated or mounted). The tray 20 is extracted from or retracted into the main body 10 through an opening which is formed at a predetermined position in a surface of the main body 10 and, more preferably, is extracted from or retracted into the main body 10 below an optical unit 70 which is installed in the main body 10. The tray 20 functions to dispose the circular cartridge 30 in the main body 10 at a position corresponding to the optical unit 70.

The tray 20 is extracted from or retracted into the main body 10 by a motor drive unit 82 in such a way that it slides along a guide rail 21 provided in the main body 10. The tray 20 may be operated in such a way that, when the user selects a retraction command or an extraction command using the touch screen panel 11, it conducts the corresponding operation. In the case where the tray 20 is in the extracted state, it may be retracted into the main body 10 in such a way that the user pushes it with his/her hand.

The tray 20 has a long rectangular planar shape. A guide depression 22 that has a predetermined depth is longitudinally formed in a lower surface of the tray 20 so that the guide rail 21 is inserted into the guide depression 22. A seating depression 23 that has a predetermined depth and has a shape corresponding to a peripheral shape of the circular cartridge 30 is formed in an end of the tray 20 so that the circular cartridge 30 is seated into the seating depression 23 of the tray 20.

The opening through which the tray 20 is extracted from or retracted into the main body 10 has a tray close contact structure such that when the tray 20 is completely retracted into the main body 10, external light is prevented from being applied onto the optical unit 70 disposed in the main body 10.

The circular cartridge 30 has a cylindrical shape and includes a multilayered porous membrane filter, a liquid absorbing layer, etc. An injection hole 31 is formed to a predetermined depth in a central portion of an upper portion of the circular cartridge 30. A sample (a mixed solution of blood and reagent) to be measured is injected into the injection hole 31. in the present invention, when the tray 20 is retracted into the main body, the sample must be disposed below the optical unit 70 through the injection hole 31 of the circular cartridge 30. As such, because the injection hole 31 is formed in the central portion of the circular cartridge 30, when it is required to seat the circular cartridge 30 into the seating depression 23 of the tray 20, the user has only to seat the circular cartridge 30 into the seating depression 23 without taking the orientation into account.

A kit depression 41 that has a predetermined depth is formed in a bottom surface of the seating depression 23 of the tray 20. A white calibration kit 40 is removably mounted into the kit depression 41. The white calibration kit 40 has a white planar shape such that it can be used in automatic calibration. Locking protrusions 42 are provided on opposite side edges of the white calibration kit 40 so that the white calibration kit 40 can be locked to the tray 20 by the locking protrusions 42. Hooks 42a protrude from ends of the respective locking protrusions 42 in directions facing each other so that the white calibration kit 40 can be maintained in a temporary locked state on the opposite edges of the tray 20. The automatic calibration operation introduced in the present invention will be explained in detail below.

The kit depression 41 has a depth corresponding to the thickness of the white calibration kit 40. When the white calibration kit 40 is disposed in the kit depression 41, an upper surface of the white calibration kit 40 is level with the bottom surface of the seating depression 23 so that the circular cartridge 30 can be seated into the seating depression 23 after the white calibration kit 40 has been disposed in the kit depression 41.

A cartridge support 50 is provided in the main body 10 at a predetermined position corresponding to a path along which the tray moves. Preferably, the cartridge support 50 elastically supports the circular cartridge 30 such that when the tray 20 is completely retracted into the main body 10, the circular cartridge 30 which has been placed on the tray 20 can be precisely disposed below the optical unit 70.

The cartridge support 50 includes pressing members 52 which are slidably provided on respectively guide rails 51 that are provided on opposite sides of the circular cartridge 30 which has been disposed below the optical unit 70 by the retraction of the tray, and springs 53 which are coupled at first ends thereof to the corresponding guide rails 51 and support at second ends thereof the corresponding pressing members 52. The springs 53 push the pressing members 52 towards the circular cartridge 30, whereby the pressing members 52 elastically support the circular cartridge 30. Here, each spring is an elastic member, and any kind of material, for example, plastic, can be used as the spring, so long as it has sufficient elasticity. Because the cartridge support 50 has the above-mentioned construction, optical measurement focusing can be precisely performed even without changing the focusing position of the optical unit 70. Therefore, the accuracy of the measurement value can be enhanced.

The optical unit 70 of the glycosylated hemoglobin measurement apparatus of the present invention will be described.

The optical unit 70 is installed in the main body 10 and disposed above the circular cartridge 30, in other words, just above the circular cartridge 30 which has been placed on the tray 20 which has been completely retracted into the main body 10. A frame 61 is mounted to a base 60 of the optical unit 70. Optical elements, for example, a photo-interrupter (a cartridge installation detection unit) 62, an LED (a light emitting part) 64 and a photo-diode (a light receiving part) 63, are mounted to the frame 61. That is, the base 60 supports the optical elements, and the frame 61 fixes the optical elements in place. The photo-interrupter 62, the LED 64 and the photo-diode 63 are mounted in different respective slots of the frame 61 such that light which is emitted from the photo-interrupter 62 and the LED 64 is prevented from entering the photo-diode 63.

The photo-interrupter 62 which is disposed in a side portion of the frame 61 includes an infrared LED and an infrared photo-diode. The photo-interrupter 62 detects whether the circular cartridge 30 has been placed on the tray 20. In this embodiment, although the photo-interrupter 62 is illustrated as being only one example of the cartridge installation detection unit, the cartridge installation detection unit may be embodied by another method.

The principle of detecting whether the cartridge is present will be explained with reference to Fig. 5. The photo-interrupter 62 is operated every time the retraction of the tray occurs. After the tray 20 has been retracted into the main body, whether the circular cartridge is present can be detected by checking light that has been emitted towards the tray 20 and reflected therefrom. In detail, if the tray 20 is retracted into the main body without the circular cartridge 30 having been placed on the tray 20, light emitted from the photo-interrupter 62 returns after being reflected by the seating depression 23 of the tray 20. If the tray 20 is retracted into the main body with the circular cartridge 30 having been placed on the tray 20, light emitted from the photo-interrupter 62 returns after being reflected by the circular cartridge 30. Therefore, whether the cartridge has been mounted to the tray can be detected by a difference between light reflected by the tray seating depression 23 and light reflected by the circular cartridge 30.

The photo-diode 63 is provided in a central portion of the frame 61. LEDs 64 are radially provided around the photo-diode 63. The LEDs 64 include two red LEDs, two blue LEDs and two green LEDs. Depending on a control of light emitted from the LEDs, the light may be used to measure glycosylated hemoglobin or used for automatic calibration. Glycosylated hemoglobin is measured by light which is applied from the optical unit to reagent injected into the circular cartridge 30 and then reflected by the reagent. The operation of measuring glycosylated hemoglobin will be explained in detail below.

The measurement apparatus of the present invention can be used to measure not only glycosylated hemoglobin but also albumin, creatinine, C-reactive protein, D-dimer, etc. This can be achieved by changing the reagent which is injected into the circular cartridge along with biomaterial (e.g., blood, urine, blood plasma, intestinal juices, cerebral fluid, spinal fluid, saliva, etc.), for example, by changing a reagent such as boronic acid, concanavalin, antibody, etc., which can be bound to glycosylated hemoglobin, and using a different reagent corresponding to a target, or by using an optical measurement method (e.g., colorimetry, luminometry, absorptiometry, fluorometry, etc.) which uses optical characteristics that correspond to the reagent and target.

As a first example, a process of measuring glycosylated hemoglobin using a new coumarin derivative, which was proposed in Korean Patent Application No. 2010-0081216 which was filed by the applicant of the present invention will be described below. In Korean Patent Application No. 2010-0081216, boronic acid which is known as having high binding force with glycosylated hemoglobin is coupled to glycosylated hemoglobin in such a way that aminophenylboronic acid is introduced to a coumarin parent that has high solubility with respect to a water solution and is fluorescent. A glycosylated hemoglobin content in blood can be measured by selective luminescence of coumarin.

In the present invention, a mixed solution (a sample) of blood and reagent produced using the above-stated coumarin derivative is prepared. By making such a mixture, aminophenylboronic acid, which is formed by binding coumarin to glycosylated hemoglobin in the blood of the sample, is created. The sample is thereafter injected into the circular cartridge 30. Subsequently, a washing solution is injected into the circular cartridge 30 so as to remove components, other than the reagent to which glycosylated hemoglobin is bound, that is, reagent that has not been bound to glycosylated hemoglobin, unglycosylated hemoglobin, etc. [by filtering out them from a top surface to a bottom surface of the circular cartridge 30]. Thereafter, the circular cartridge 30 is placed onto the tray 20 and inserted into the main body 10. The optical unit 70 thereafter applies light onto the sample of the circular cartridge 30. Then, glycosylated hemoglobin content can be measured by fluorescent light emitted from coumarin of the reagent to which glycosylated hemoglobin is bound.

As a second example, a process of measuring glycosylated hemoglobin using reflectivity of light with a widely known boronic acid derivative reagent will be described below.

In the glycosylated hemoglobin measurement apparatus of the present invention, after the circular cartridge 30, into which a mixed sample of blood and boronic acid derivative reagent has been injected, is placed on the tray 20 and inserted into the main body 10, the red LEDs of the optical unit 70 apply light onto the sample, and the intensity of light reflected by the sample is measured. Further, the blue LEDs of the optical unit 70 apply light onto the sample, and the intensity of light reflected by the sample is measured. A measurement value[%HbA1c = glycosylated haemoglobin / total haemoglobin] which is expressed by a ratio of glycosylated haemoglobin to total haemoglobin is calculated from a ratio of the intensity of light resulting from the blue LEDs to the intensity of light resulting from the red LEDs [%HbA1c = red color intensity / blue color intensity].

The operation of the automatic calibration introduced in the present invention will be explained in detail below.

The optical measurement apparatus uses optical elements such as LEDs, etc. Due to characteristics of the optical elements, it is difficult for the optical elements to emit the one hundred percent quantity of light every time the measurement is conducted. Depending on variation in power, etc., the quantity of light is slightly changed. In addition, the quantity of light may vary depending on the remaining lifespan of the optical elements. Therefore, calibration is required to compensate for a deviation in the quantity of light.

For reference, calibration in the conventional glycosylated hemoglobin measurement apparatus using a pen type light source will be explained. The user must place a light source unit, provided in a lower end of a pen, on a white plate, every time glycosylated haemoglobin measurement is conducted. Thereafter, the light source unit applies light onto the white plate, and the calibration is carried out using light reflected by the white plate. However, this conventional technique provides a manual calibration method which requires the user handling the calibration process himself/herself. It is very difficult to precisely conduct the calibration because of the hand of the user may be unsteady. Furthermore, because the user directly holds the light source unit with his/her hand, the deviation in the result of calibration is increased, thus making precise calibration difficult.

To prevent these problems, the present invention provides an automatic calibration function in addition to the structure including the white calibration kit 40 as an automatic calibration kit, and the tray onto which the white calibration kit 40 is placed. The automatic calibration operation of the present invention is conducted when the tray 20 is retracted into the main body, in other words, the tray 20 that has been extracted from the main body is retracted thereinto after power is turned on [or, after measurement of glycosylated haemoglobin has been completed and the circular cartridge has been removed, when the tray is retracted into the main body to conduct subsequent measurement operation]. Preferably, the automatic calibration operation of the present invention is conducted along with a process of detecting whether the circular cartridge is present on the tray.

In detail, the white calibration kit 40 is provided with locking members. The kit depression 41 is formed in the seating depression 23 of the tray 20 so that the white calibration kit 40 can be mounted to the tray 20. Particularly, the present invention is configured such that after the white calibration kit 40 has been mounted to the tray 20, the circular cartridge 30 can be placed into the seating depression 23 of the tray 20 above the white calibration kit 40.

The white calibration kit 40 is removably mounted to the seating depression 23 of the tray 20. The reason for this is due to the fact that if contaminants are applied to the surface of the white calibration kit 40 while glycosylated haemoglobin is being measured, or the reflectivity is reduced over a long period of time, it must be possible to replace the white calibration kit 40 with a new one.

The automatic calibration operation of the present invention will now be explained. When the tray 20 is retracted into the main body, the photo-interrupter 62 detects whether the circular cartridge 30 has been mounted to the tray 20. If the circular cartridge 30 is present on the tray 20, this indicates that the glycosylated haemoglobin measurement operation will be carried out, so the automatic calibration operation is not conducted. If the circular cartridge 30 is not on the tray 20, the automatic calibration operation is carried out. In detail, the red LEDs, the green LEDs and the blue LEDs of the optical unit 70 emit light towards the white calibration kit 40 which is disposed in the seating depression 23 of the tray 20 and, thereafter, the optical unit 70 receives light reflected by the white calibration kit 40 and checks a deviation with respect to the reference quantity of light which has been stored in the optical unit 70. The checked deviation in the quantity of light is used to compensate for the quantity of light with regard to a measurement value of glycosylated haemoglobin in a subsequent glycosylated haemoglobin measurement process. As such, it must be understood that the automatic calibration refers to compensating for the measurement value using the deviation in the quantity of light. In the present invention, if the checked deviation in the quantity of light is beyond a preset range, a subsequent glycosylated haemoglobin measurement process is no longer progressed, and the user is notified of the necessary replacement of the corresponding element of the optical unit.

The present invention having the above-mentioned construction does not require separately having an optical unit which is used to measure glycosylated haemoglobin and an optical unit which is used for automatic calibration. Therefore, the present invention can carry out both the glycosylated haemoglobin measurement and the automatic calibration without an additional optical element. Furthermore, before every glycosylated haemoglobin measurement, the automatic calibration is automatically performed. Therefore, a measurement value of glycosylated haemoglobin can be more precisely obtained.

Meanwhile, the glycosylated haemoglobin measurement requires an operation of mixing blood with reagent, an operation of incubating the mixed sample, an operation of injecting the incubated sample into the circular cartridge 30, an operation of injecting a washing solution into the circular cartridge 30, an operation of placing the circular cartridge 30 onto the tray 20 of the glycosylated hemoglobin measurement apparatus and inserting it into the main body of the apparatus, etc. However, it is not easy for a novice or any ordinary person other than an expert to perform such operations in preparing a sample. Moreover, the order in which the sample preparation operations are performed, and the timing of each operation, affect the measurement value.

Due to these reasons, the present invention provides a function of guiding the sample preparation operations.

The user can successively carry out the sample preparation operations at the correct times according to the guide of the glycosylated hemoglobin measurement apparatus. The sample preparation process guide function is performed by a screen displayed on the screen panel 11 of the glycosylated hemoglobin measurement apparatus and/or voices coming from a speaker 81 [in a guide mode]. Of course, after the sample preparation process has been conducted, an expert may select a manual mode from the menu and carry out operation of placing the circular cartridge 30 onto the tray 20 of the glycosylated hemoglobin measurement apparatus and inserting it into the main body.

For example, when the user mixes blood with reagent and selects a Next button, as shown in Fig. 9, the amount of time it takes to conduct the incubation operation is indicated as two minutes on a screen to instruct the user to stand by during the incubation. When the incubation is completed, an 'Apply Sample' screen is expressed to instruct the user to inject the incubated sample into the circular cartridge 30. After fifteen seconds, [in this case, fifteen seconds are a time period determined by the time it generally takes for the user to inject the sample into the cartridge], an 'Apply Washing' screen is displayed to instruct the user to inject the washing solution into the circular cartridge 30. After a further fifteen seconds, [in this case, fifteen seconds are a time period determined by the time it generally takes for the user to inject the washing solution into the cartridge], an 'Insert Cartridge' screen is displayed to instruct the user to place the circular cartridge 30 onto the tray 20 of the glycosylated hemoglobin measurement apparatus and insert it into the main body.

As stated above, the present invention makes it possible for the user to correctly conduct the sample preparation process, thus prevent expensive accessories (reagent, etc.) from being discarded without being properly used. Furthermore, the present invention guides the user so that he/she can carry out the correct sample preparation process, thus preventing the glycosylated hemoglobin measurement apparatus from making an incorrect measurement value.

The operation of the glycosylated hemoglobin measurement apparatus according to the embodiment of the present invention will be described in detail with reference to Fig. 10.

To begin glycosylated hemoglobin measurement, the user turns on the glycosylated hemoglobin measurement apparatus. Here, it is preferable that the circular cartridge 30 has not been placed on the tray 20 of the glycosylated hemoglobin measurement apparatus but the white calibration kit 40 has been mounted to the tray 20.

Then, the glycosylated hemoglobin measurement apparatus begins to conduct automatic calibration. That is, the LEDs 64 [including the red LEDs, the green LEDs and the blue LEDs] of the optical unit 70 apply light onto the white calibration kit 40, and the photo-diode 63 receives light reflected by the white calibration kit 40. Thereafter, the glycosylated hemoglobin measurement apparatus checks a deviation between the quantity of light received from the white calibration kit 40 and the reference quantity of light of the optical unit. Of course, before the automatic calibration begins, the glycosylated hemoglobin measurement apparatus preferably detects using the photo-interrupter 62 whether the circular cartridge 30 has been present on the tray 20

After the automatic calibration has been completed, the glycosylated hemoglobin measurement apparatus displays a mode selection menu on the touch screen panel 11 so that the user can select either the guide mode or the manual mode.

If the user selects the guide mode, the sample preparation operation guide process which has been illustrated with reference to Fig. 9 is conducted. If the user selects the manual mode, the user extracts the tray 20 from the main body to enable the circular cartridge 30 to be placed onto the tray 20, after having conducted the sample preparation process.

While the user seats the circular cartridge 30 into the seating depression 23 of the tray 20 and inserts the tray 20 into the main body, the circular cartridge 30 is maintained in the state of having been fixed between the pressing members 52. When the tray 20 is inserted into the main body, the injection hole 31 formed in the central portion of the circular cartridge 30 is disposed at a position corresponding to the focusing points of the LEDs 64 and the photo-diode 63. As such, since the injection hole 31 is formed in the central portion of the circular cartridge 30, the injection hole 31 can be disposed at a position correspond to the focusing points of the LEDs 64 and the photo-diode 63 at the same time as the insertion of the tray 20, regardless of the orientation of the circular cartridge 30.

After the tray 20 has been completely inserted into the main body, the glycosylated hemoglobin measurement begins. That is, as described above, the optical unit 70 applies light to the sample that has been in the circular cartridge 30 and receives light reflected therefrom, thus measuring glycosylated hemoglobin content. Of course, the glycosylated hemoglobin measurement apparatus preferably conducts the operation of detecting whether the circular cartridge 30 has been placed on the tray 20 using the photo-interrupter 62 before the glycosylated hemoglobin measurement.

Subsequently, the glycosylated hemoglobin measurement apparatus applies the checked deviation in the quantity of light to the measurement value of glycosylated hemoglobin, thus compensating for the measurement value. Thereafter, the glycosylated hemoglobin measurement apparatus outputs the measurement value of glycosylated hemoglobin through the touch screen panel 11 and/or the speaker 81.

Although the preferred embodiment of the present invention has been disclosed for illustrative purposes, those skilled in the art will appreciate that various modifications, additions and substitutions are possible, without departing from the scope of the invention as disclosed in the accompanying claims.

## Claims

1. An optical measurement apparatus, comprising:
an optical unit (70); and
a tray (20) extracted from a main body (10) through an opening formed in a predetermined portion of the main body (10) or retracted into the main body through the opening towards an optical unit (70) provided in the main body (10), wherein a cartridge (30) into which a sample is injected is seated onto the tray (20);
a cartridge support (50) fixing the cartridge (30) at a position corresponding to the optical unit (70) after the cartridge (30) has been seated on the tray (20) and the tray has been retracted into the main body (10);
the optical unit (70) applying light to the sample contained in the fixed cartridge (30) and receiving light from the sample; and
a control unit calculating a measurement value of the sample using the applied light and the received light; and wherein the tray has a seating depression (23) into which the cartridge is seated, and further comprising
a cartridge installation detection unit detecting whether the cartridge (30) has been mounted on the tray when the tray (20) is in the retracted state, and
wherein the cartridge installation detection unit detects whether the cartridge (30) has been mounted to the tray (20) by detecting a difference between light reflected by the tray seating depression (23) and light reflected by the cartridge (30); wherein the seating depression (23) has a predetermined depth and has a shape corresponding to a peripheral shape of the cartridge (30);
wherein the cartridge (30) has a circular shape, with a sample injection hole (31) formed in a central portion of one surface of the cartridge (30); and wherein the cartridge support comprises:
pressing members (52) slidably provided on opposite sides of the circular cartridge (30) which has been disposed below the optical unit (70) by the retraction of the tray (20); and
elastic members supporting the corresponding pressing members (52) and pressing the pressing members towards the cartridge (30).

2. The optical measurement apparatus according to claim 1, wherein a kit depression (41) is formed in a bottom surface of the tray seating depression (23), and a white calibration kit (40) is removably mounted into the kit depression (41), the white calibration kit (40) being used to check a deviation in the quantity of light of the optical unit (70).

3. The optical measurement apparatus according to claim 2, wherein the white calibration kit (40) has a white plate shape, with locking protrusions (42) provided on opposite edges of the white calibration kit (40), each of the locking protrusions (42) having a hook (42a) so that the locking protrusions are locked to the tray, or wherein an upper surface of the white calibration kit (40) mounted in the kit depression (41) is level with the bottom surface of the tray seating depression (23) so that the cartridge (30) can be seated onto the white calibration kit (40) in the tray seating depression (23).

4. The optical measurement apparatus according to claim 1, wherein the control unit guides a user conducting a sample preparation operation through a screen and/or a voice.

5. The optical measurement apparatus according to claim 1, wherein the optical unit comprises:
a light emitting part (64) applying light to the sample contained in the fixed cartridge; and
a light receiving part (63) receiving light reflected from the sample by the light application of the light emitting part (64).

6. The optical measurement apparatus according to claim 5, wherein the light emitting part (64) and the light receiving part (63) of the optical unit (70) are installed in respective slots formed in a frame.

7. The optical measurement apparatus according to claim 6, wherein the light receiving part (63) is installed in a central portion of the frame, and the light emitting part (64) comprises a plurality of light sources arranged around the central portion of the frame.

8. The optical measurement apparatus according to claim 7, wherein, when the tray (20) is completely retracted into the main body (10), a sample injection hole (31) of the cartridge (30) seated on the tray (20) is disposed below the optical unit of the frame.

9. The optical measurement apparatus according to claim 1, wherein the control unit calculates the measurement value of the sample when the cartridge installation detection unit detects a presence of the cartridge (30).

10. The optical measurement apparatus according to claim 2, wherein when the cartridge (30) has not been mounted on the tray (20) and power is turned on, the control unit checks the deviation.

11. The optical measurement apparatus according to claim 2, wherein when the tray that has been extracted from the main body is retracted thereinto without the cartridge (30) having been mounted, the control unit checks the deviation.

## Patentansprüche

1. Optische Messeinrichtung, umfassend:
eine optische Einheit (70); und
einen Einsatz (20), aus einem Hauptkörper (10) durch eine Öffnung herausgezogen, die in einem vorher festgelegten Bereich des Hauptkörpers (10) gebildet ist, oder durch die Öffnung in Richtung einer optischen Einheit (70) in den Hauptkörper zurückgezogen, die in dem Hauptkörper (10) bereitgestellt ist, wobei eine Kartusche (30), in die eine Probe eingespritzt ist, auf den Einsatz (20) gesetzt wird;
einen Kartuschenträger (50), der die Kartusche (30) an einer Position fixiert, die der optischen Einheit (70) entspricht, nachdem die Kartusche (30) auf den Einsatz (20) gesetzt wurde und der Einsatz in den Hauptkörper (10) zurückgezogen wurde;
wobei die optische Einheit (70) Licht auf die in der fixierten Kartusche (30) enthaltene Probe anwendet und Licht von der Probe empfängt; und
wobei eine Steuerungseinheit einen Messwert der Probe unter Verwendung des angewendeten Lichts und des empfangenen Lichts berechnet; und wobei der Einsatz eine Sitzvertiefung (23) aufweist, in die die Kartusche gesetzt wird, und weiter umfassend
eine Kartuscheninstallation-Detektionseinheit, die detektiert, ob die Kartusche (30) auf dem Einsatz montiert wurde, wenn der Einsatz (20) in dem zurückgezogenen Zustand vorliegt, und wobei die Kartuscheninstallation-Detektionseinheit detektiert, ob die Kartusche (30) an dem Einsatz (20) montiert wurde, indem eine Differenz zwischen Licht, das von der Einsatzsitzvertiefung (23) reflektiert wird, und Licht, das von der Kartusche (30) reflektiert wird, detektiert wird; wobei die Sitzvertiefung (23) eine vorher festgelegte Tiefe aufweist und eine Form entsprechend einer Umfangsform der Kartusche (30) aufweist;
wobei die Kartusche (30) eine kreisförmige Form mit einem in einem zentralen Bereich gebildeten Probeneinspritzloch (31) auf einer Oberfläche der Kartusche (30) aufweist; und wobei der Kartuschenträger umfasst:
Andrückelemente (52), gleitend auf gegenüberliegenden Seiten der kreisförmigen Kartusche (30) bereitgestellt, die durch das Zurückziehen des Einsatzes (20) unterhalb der optischen Einheit (70) angeordnet wurde; und
elastische Elemente, die die entsprechenden Andrückelemente (52) tragen und die Andrückelemente in Richtung der Kartusche (30) drücken.

2. Optische Messeinrichtung nach Anspruch 1, wobei eine Kitvertiefung (41) in einer unteren Oberfläche der Einsatzsitzvertiefung (23) gebildet ist und ein weißes Kalibrierungskit (40) entfernbar in die Kitvertiefung (41) montiert ist, wobei das weiße Kalibrierungskit (40) verwendet wird, um eine Abweichung in der Menge an Licht von der optischen Einheit (70) zu überprüfen.

3. Optische Messeinrichtung nach Anspruch 2, wobei das weiße Kalibrierungskit (40) eine weiße Plattenform mit Verriegelungsvorsprüngen (42) aufweist, die an gegenüberliegenden Rändern des weißen Kalibrierungskits (40) bereitgestellt sind, wobei jeder der Verriegelungsvorsprünge (42) einen Haken (42a) aufweist, sodass die Verriegelungsvorsprünge an dem Einsatz verriegelt werden, oder wobei eine obere Oberfläche des weißen Kalibrierungskits (40), das in der Kitvertiefung (41) montiert ist, mit der unteren Oberfläche der Einsatzsitzvertiefung (23) bündig ist, sodass die Kartusche (30) auf das weiße Kalibrierungskit (40) in der Einsatzsitzvertiefung (23) gesetzt werden kann.

4. Optische Messeinrichtung nach Anspruch 1, wobei die Steuerungseinheit einen Anwender, der einen Probenvorbereitungsvorgang ausführt, durch einen Bildschirm und/oder eine Stimme anleitet.

5. Optische Messeinrichtung nach Anspruch 1, wobei die optische Einheit umfasst:
ein lichtemittierendes Teil (64), das Licht auf die in der fixierten Kartusche enthaltene Probe anwendet; und
ein lichtempfangendes Teil (63), das Licht von der Lichtanwendung des lichtemittierenden Teils (64), das von der Probe reflektiert wurde, empfängt.

6. Optische Messeinrichtung nach Anspruch 5, wobei das lichtemittierende Teil (64) und das lichtempfangende Teil (63) der optischen Einheit (70) in jeweiligen in einem Rahmen gebildeten Schlitzen installiert sind.

7. Optische Messeinrichtung nach Anspruch 6, wobei das lichtempfangende Teil (63) in einem zentralen Bereich des Rahmens installiert ist und das lichtemittierende Teil (64) eine Vielzahl von Lichtquellen umfasst, die um den zentralen Bereich des Rahmens herum angebracht sind.

8. Optische Messeinrichtung nach Anspruch 7, wobei, wenn der Einsatz (20) vollständig in den Hauptkörper (10) zurückgezogen ist, ein Probeneinspritzloch (31) der auf den Einsatz (20) gesetzten Kartusche (30) unterhalb der optischen Einheit des Rahmens angeordnet ist.

9. Optische Messeinrichtung nach Anspruch 1, wobei die Steuerungseinheit den Messwert der Probe berechnet, wenn die Kartuscheninstallation-Detektionseinheit ein Vorhandensein der Kartusche (30) detektiert.

10. Optische Messeinrichtung nach Anspruch 2, wobei, wenn die Kartusche (30) nicht an dem Einsatz (20) montiert ist und Netzstrom eingeschaltet ist, die Steuerungseinheit die Abweichung überprüft.

11. Optische Messeinrichtung nach Anspruch 2, wobei, wenn der Einsatz, der aus dem Hauptkörper herausgezogen wurde, dort hinein zurückgezogen wird, ohne dass die Kartusche (30) montiert worden ist, die Steuerungseinheit die Abweichung überprüft.

## Revendications

1. Appareil de mesure optique, comprenant :
une unité optique (70) ; et
un plateau (20) extrait à partir d'un corps principal (10) à travers une ouverture formée dans une partie prédéterminée du corps principal (10) ou rétracté dans le corps principal à travers l'ouverture vers une unité optique (70) fournie dans le corps principal (10), dans lequel une cartouche (30) dans laquelle un échantillon est injecté est installée sur le plateau (20) ;
un support de cartouche (50) fixant la cartouche (30) à une position correspondant à l'unité optique (70) après que la cartouche (30) a été installée sur le plateau (20) et que le plateau a été rétracté dans le corps principal (10) ;
l'unité optique (70) appliquant une lumière sur l'échantillon contenu dans la cartouche (30) fixée et recevant une lumière provenant de l'échantillon ; et
une unité de commande calculant une valeur de mesure de l'échantillon en utilisant la lumière appliquée et la lumière reçue ; et dans lequel le plateau présente une dépression d'installation (23) dans laquelle la cartouche est installée, et comprenant en outre
une unité de détection d'installation de cartouche détectant si la cartouche (30) a été montée sur le plateau quand le plateau (20) est dans l'état rétracté, et dans lequel l'unité de détection d'installation de cartouche détecte si la cartouche (30) a été montée sur le plateau (20) en détectant une différence entre la lumière réfléchie par la dépression d'installation (23) du plateau et la lumière réfléchie par la cartouche (30) ; dans lequel la dépression d'installation (23) présente une profondeur prédéterminée et présente une forme correspondant à une forme périphérique de la cartouche (30) ;
dans lequel la cartouche (30) présente une forme circulaire, avec un trou d'injection d'échantillon (31) formé dans une partie centrale d'une surface de la cartouche (30) ; et dans lequel le support de cartouche comprend :
des éléments presseurs (52) fournis de manière coulissante sur les côtés opposés de la cartouche circulaire (30) qui a été disposée sous l'unité optique (70) par la rétraction du plateau (20) ; et
des éléments élastiques supportant les éléments presseurs (52) correspondants et pressant les éléments presseurs vers la cartouche (30).

2. Appareil de mesure optique selon la revendication 1, dans lequel une dépression pour kit (41) est formée dans une surface inférieure de la dépression d'installation (23) du plateau, et un kit d'étalonnage à blanc (40) est monté de manière amovible dans la dépression pour kit (41), le kit d'étalonnage à blanc (40) étant utilisé pour vérifier une déviation de la quantité de lumière de l'unité optique (70).

3. Appareil de mesure optique selon la revendication 2, dans lequel le kit d'étalonnage à blanc (40) présente une forme de plaque blanche, avec des saillies de verrouillage (42) fournies sur les bords opposés du kit d'étalonnage à blanc (40), chacune des saillies de verrouillage (42) présentant un crochet (42a) de sorte que les saillies de verrouillage sont verrouillées sur le plateau, ou dans lequel une surface supérieure du kit d'étalonnage à blanc (40) monté dans la dépression pour kit (41) est au niveau de la surface inférieure de la dépression d'installation (23) du plateau de sorte que la cartouche (30) peut être installée sur le kit d'étalonnage à blanc (40) dans la dépression d'installation (23) du plateau.

4. Appareil de mesure optique selon la revendication 1, dans lequel l'unité de commande guide un utilisateur effectuant une opération de préparation d'échantillon au moyen d'un écran et/ou d'une voix.

5. Appareil de mesure optique selon la revendication 1, dans lequel l'unité optique comprend :
une partie émettrice de lumière (64) appliquant une lumière sur l'échantillon contenu dans la cartouche fixée ; et
une partie réceptrice de lumière (63) recevant une lumière réfléchie par l'échantillon par l'application de lumière de la partie émettrice de lumière (64).

6. Appareil de mesure optique selon la revendication 5, dans lequel la partie émettrice de lumière (64) et la partie réceptrice de lumière (63) de l'unité optique (70) sont installées dans des fentes respectives formées dans un cadre.

7. Appareil de mesure optique selon la revendication 6, dans lequel la partie réceptrice de lumière (63) est installée dans une partie centrale du cadre, et la partie émettrice de lumière (64) comprend une pluralité de sources de lumière agencées autour de la partie centrale du cadre.

8. Appareil de mesure optique selon la revendication 7, dans lequel, quand le plateau (20) est complètement rétracté dans le corps principal (10), un trou d'injection d'échantillon (31) de la cartouche (30) installée sur le plateau (20) est disposé sous l'unité optique du cadre.

9. Appareil de mesure optique selon la revendication 1, dans lequel l'unité de commande calcule la valeur de mesure de l'échantillon quand l'unité de détection d'installation de cartouche détecte une présence de la cartouche (30).

10. Appareil de mesure optique selon la revendication 2, dans lequel quand la cartouche (30) n'a pas été montée sur le plateau (20) et que l'alimentation est activée, l'unité de commande vérifie la déviation.

11. Appareil de mesure optique selon la revendication 2, dans lequel quand le plateau qui a été extrait à partir du corps principal est rétracté dans celui-ci sans que la cartouche (30) ait été montée, l'unité de commande vérifie la déviation.
